(19) Europäisches Patentamt
European Patent Office
Office européen des brevets

(11) **EP 2 648 113 A1**

(12) **EUROPEAN PATENT APPLICATION**
published in accordance with Art. 153(4) EPC

(43) Date of publication:
**09.10.2013 Bulletin 2013/41**

(51) Int Cl.:
***G06F 17/21*** *(2006.01)*

(21) Application number: **11845632.6**

(22) Date of filing: **02.12.2011**

(86) International application number:
**PCT/CN2011/083394**

(87) International publication number:
**WO 2012/072043 (07.06.2012 Gazette 2012/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.12.2010  CN 201010578118**

(71) Applicants:
• **Peking University Founder Group Co., Ltd**
  **Haidian District**
  **Beijing**
  **100871 (CN)**

• **Beijing Founder Electronics Co., Ltd.**
  **Haidian District, Beijing 100085 (CN)**

(72) Inventor: **ZHAO, Zhigang**
  **Beijing 100085 (CN)**

(74) Representative: **Algemeen Octrooi- en Merkenbureau B.V.**
  **P.O. Box 645**
  **5600 AP Eindhoven (NL)**

(54) **THE METHOD FOR ADJUSTING ANGLE OF CHEMICAL BOND FIGURE AND DEVICE THEREOF**

(57)     The application provides a method for adjusting angles between graphical chemical bond vectors comprising: receiving an input from specific keys of a keyboard when a typesetting focus is located at one end of a graphical chemical bond vector; determining a rotating direction for the vector based on the received input; setting a rotating angle for the graphical chemical bond vector in the determined rotating direction; and rotating the graphical chemical bond vector about the other end thereof by the set rotating angle in the determined rotating direction. The application further provides an apparatus for adjusting angles between graphical chemical bond vectors comprising: an inputting module configured to receive an input from specific keys of a keyboard when a typesetting focus is located at one end of a graphical chemical bond vector; a direction module configured to determine a rotating direction for the vector based on the received input; an angle module configured to set a rotating angle for the graphical chemical bond vector in the determined rotating direction; and a rotating module configured to rotate the graphical chemical bond vector about the other end thereof by the set rotating angle in the determined rotating direction. According to the present application, operation efficiency of adjustment for graphical chemical bond vectors can be improved.

FIG.3

## Description

## Technical Field

The present application relates to a field of digital typesetting, in particular, to a method and an apparatus for adjusting angles between graphical chemical bond vectors.

## Background

Recently, chemical bonds are provided by using vector graphs in an interactive chemical typesetting software. Angles between graphical chemical bond vectors are usually required to be adjusted when a user typesets the graphical chemical bond vectors.

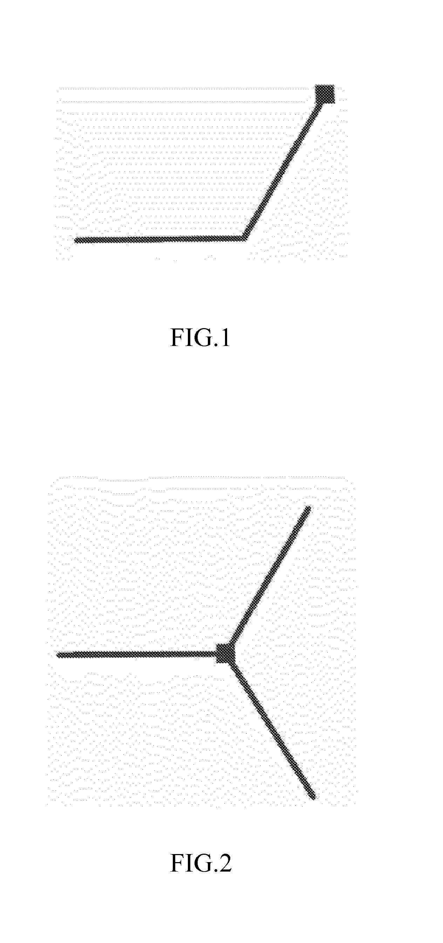

In the existing interactive chemical typesetting software, angle adjustments for the graphical chemical bond vectors are usually performed by operations of a mouse. Fig. 1 is a state diagram illustrating a selected end of a single graphical chemical bond vector, and Fig. 2 is a state diagram illustrating a selected end connected with a plurality of graphical chemical bond vectors. As shown in Figs. 1 and 2, in the interactive chemical typesetting software, the user moves the mouse, which in turn moves a focus of the vector so as to change angles formed between the graphical chemical bond vectors.

However, in typesetting chemical contents, graphical chemical bond vectors are usually required to compose a regular polygon. The inventors found that angle adjustments for graphical chemical bond vectors are complicated and it is difficult to obtain the regular polygon due to uncontrollable degrees of freedom of the mouse in the existing operations of the mouse.

## Summary

The present application intends to provide a method and an apparatus for adjusting angles between graphical chemical bond vectors at least in light of complicated operations of a mouse in the prior art.

According embodiments of the present application, a method for adjusting angles between graphical chemical bond vectors is provided. The method comprises:

receiving an input from specific keys of a keyboard when a typesetting focus is located at one end of a graphical chemical bond vector;
determining a rotating direction for the vector based on the received input;
setting a rotating angle for the vector in the determined rotating direction; and
rotating the vector about the other end thereof by the set rotating angle and in the determined rotating direction.

According embodiments of the present application, an apparatus for adjusting angles between graphical chemical bond vectors is provided. The apparatus comprises:

an inputting module configured to receive an input from specific keys of a keyboard when an typesetting focus is located at one end of a graphical chemical bond vector;
a direction module configured to determine a rotating direction for the graphical chemical bond vector based on the received input;
an angle module configured to set a rotating angle for the graphical chemical bond vector in the determined rotating direction; and
a rotating module configured to rotate the graphical chemical bond vector about the other end thereof by the set rotating angle and in the determined rotating direction.

According to the method and the apparatus provided in embodiments of the present application, complicated adjustment operations in the prior art may be simplified and operation efficiency of adjustment for graphical chemical bond vectors may be improved by using the keyboard.

## Brief Description of the Drawing

The drawings described herein are used to provide a further understanding to the present application and constitute a part of this specification. Exemplary embodiments of the present application and their descriptions serve to explain the present application and do not constitute improper limitations on the present application. In the drawings:

Fig. 1 is a state diagram illustrating a selected end of a single chemical bond vector.

Fig. 2 is a state diagram illustrating a selected end of a plurality of connected chemical bond vectors.

Fig. 3 is a flowchart illustrating a method for adjusting angles between graphical chemical bond vectors according to an embodiment of the present application.

Fig. 4 is a schematic diagram of angle state of regular triangle to regular octagon.

Figs. 5 and 6 are state diagrams of prompt angles according to a preferred embodiment of the present application.

Fig. 7 is a schematic diagram illustrating an apparatus for adjusting angles between graphical chemical bond vectors according to an embodiment of the present application.

## Detailed Description

Hereinafter, the present application will be explained in detail with reference to the accompanying drawings in connection with embodiments thereof.

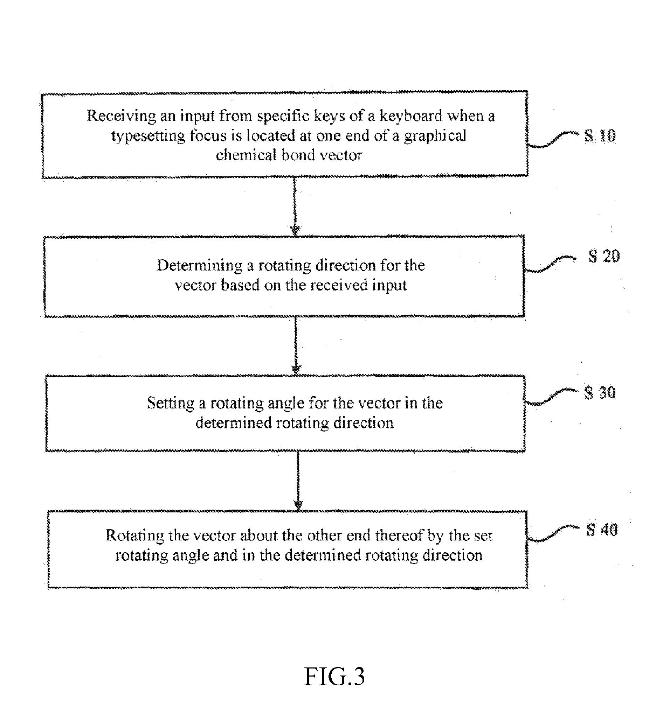

Fig. 3 is a flowchart illustrating a method for adjusting angles between graphical chemical bond vectors according to an embodiment of the present application. As shown, the method comprises:

A step S10, in which an input will be received from specific keys of a keyboard when a typesetting focus is located at one end of a graphical chemical bond vector;

A step S20, in which a rotating direction will be determined based on the received input;

A step S30, in which a rotating angle for the graphical chemical bond vector will be set in the determined rotating direction; and

A step S40 in which the graphical chemical bond vector is rotated about the other end thereof by the set rotating angle and in the determined rotating direction.

In the prior art, angles between the graphical chemical bond vectors may only be adjusted through mouse operations. However, in the present embodiment, the graphical chemical bond vectors are adjusted according to the input from the keyboard, so that efficiency of the interactive chemical typesetting software can be improved by operations of keyboard, which does not need the mouse operations to find operating types of a large number of various graphical chemical bond vectors and omits buttons for operations. And thus it facilitates the user in obtaining various expected structures, so that usability and efficiency of the chemical typesetting can be improved.

It shall be appreciated that the selected focus may be analyzed in advance. To be specific, only when one end of the graphical chemical bond vector is selected, it is possible that the graphical chemical bond vector may be rotated around the other end thereof; otherwise, a translation operation may be performed to the graphical chemical bond vector.

Preferably, in the step S20, it is determined that the received input is received from a first key, and then it is determined that the rotating direction is clockwise; or it is determined that the received input is received from a second key, and then it is determined that the rotating direction is anticlockwise. For example, the anticlockwise direction may be determined by an input received from a left cursor key "←", while the clockwise direction may be determined by an input from a right cursor key "→", which enable users to rotate the chemical bonds by clicking such keys easily.

Preferably, in step S20, it is determined that the received input is received from a first key and angle to be adjusted for the graphical chemical bond vector is within a threshold range, and then it is determined that the rotating direction is clockwise; it is determined that the received input is received from the first key and the angle to be adjusted for the graphical chemical bond vector is beyond the threshold range, and then it is determined that the rotating direction is anticlockwise; it is determined that the received input is received from a second key and the angle to be adjusted for the graphical chemical bond vector is within a threshold range, and then it is determined that the rotating direction is anticlockwise; and it is determined that the received input is received from the second key and the angle to be adjusted for the graphical chemical bond vector is beyond the threshold range, and then it is determined that the rotating direction is clockwise.

In some cases, whether or not the rotating direction is clockwise or anticlockwise may be misjudged by some users. For example, when rotating a chemical bond vector from the direction of one o'clock to two o'clock and from the direction of seven o'clock to eight o'clock, respectively, some users may click keys "→" and "←' respectively due to determination of two different directions. However, in this preferred embodiment, for example, the first and fourth quadrants may be set to be the threshold range. Then, key "→" may be set to serve for the clockwise rotation and key "←" may be set to serve for anticlockwise rotation when graphical chemical bond vectors are in the first and fourth quadrants; while key "→" may be set to serve for anticlockwise rotation and key "←" may be set to serve for clockwise rotation when graphical chemical bond vectors are in the second and third quadrants (i.e., beyond the threshold range). As another example, the first and second quadrants may be set to be the threshold range. Then, key "↓" may be set to serve for clockwise rotation and key "↑" may be set to serve for anticlockwise rotation when graphical chemical bond vectors are in the first and second quadrants; while key "↓" may be set to serve for anticlockwise rotation and key "↑" may be set to serve for clockwise rotation when graphical chemical bond vectors are in the third and fourth quadrants (i.e., beyond the threshold range).

Preferably, in step S30, if the end at which the typesetting focus is located is connected to ends of other graphical chemical bond vectors, a minimum unit (such as 1°) for rotation will be set based on each received input. Angles between the vectors and lengths of the vectors which are required to keep connection between all the connected vectors will be set as well.

As shown in Fig. 2, in the case that the typesetting focus is located at an end shared by a plurality of graphical chemical bond vectors, adjustment to an angle of graphical chemical bond vector will have an impact on other graphical chemical bond vectors. According to this preferred embodiment, the angle to be adjusted based on each keying operation is set as the minimum unit for rotation and angles and lengths of other vectors connected to the graphical chemical bond vectors are adjusted automatically to keep connection relationship therebetween, which enables the user to implement a stepless adjustment by keyboard so that fine angle adjustments for the graphical chemical bond vectors can be achieved.

Preferably, in step S30, if the end at which the typesetting focus is located is not connected to other graphical chemical bond vectors and a current angle between the graphical chemical bond vector and an adjacent graphical chemical bond vector is within an angle range, the rotation angle based on each received input is set by rule of

$$\theta = \alpha - \beta,$$

where $\alpha$ is an interior angle of a regular N- sided polygon consisted of: 1) a graphical chemical bond vector which is formed by rotating the graphical chemical bond vector by a minimum angle in the rotating direction, and 2) its adjacent graphical chemical bond vector, and $\beta$ is the current angle between the rotated graphical chemical bond vector and the adjacent graphical chemical bond vector.

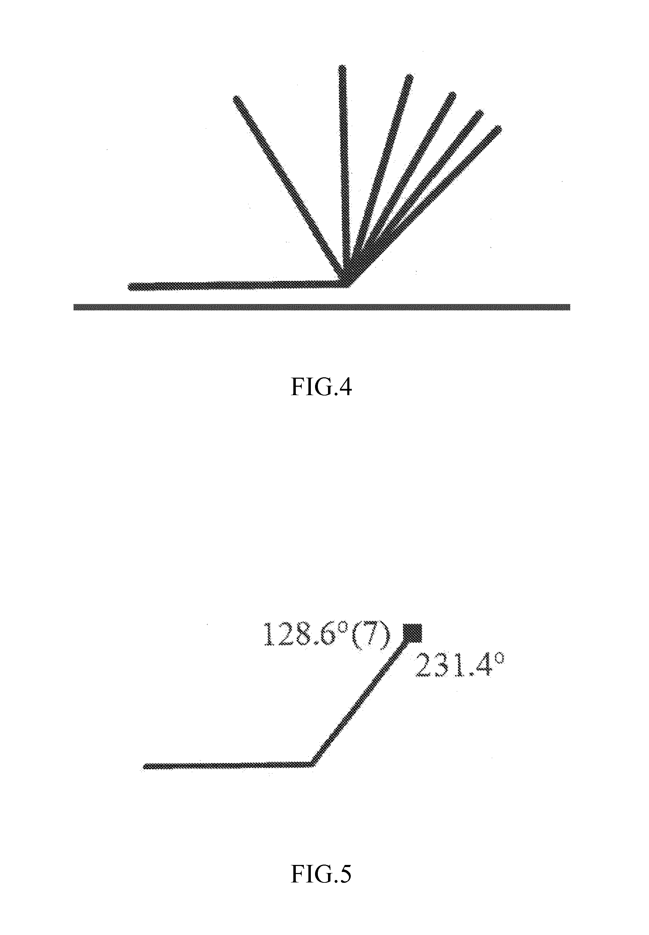

Fig. 4 is a schematic diagram of angle state of regular triangle to regular octagon. In actual chemical typesetting software, graphical chemical bond vectors are needed to be connected sequentially to compose a regular polygon (usually being a regular triangle ... a regular octagon, even a regular 10-sided polygon). Thus, when the angle between one graphical chemical bond vector to be rotated and its adjacent graphical chemical bond vector is, for example, within the range from interior angle of the regular pentagon to that of the regular hexagon, and the rotating direction is to be increased, it is expected that the user will adjust the graphical chemical bond vector so that itself and its adjacent graphical chemical bond vector compose the regular hexagon (rather than a regular heptagon) or close to the regular hexagon. In this case, according to preferred embodiment, since the angle by which the graphical chemical bond vector should be rotated in the rotating direction to compose the regular hexagon with the adjacent bond is the minimum, the rotating angle is set to be the minimum angle.

This preferred embodiment provides a step-adjustment mode, in which a certain angle (not merely 1°) is rotated rapidly each time by detecting an angle of a molecular ring intelligently. Therefore, times of operation on keyboard by the user can be decreased significantly and the regular polygon can be composed standardly.

Preferably, when there are a plurality of graphical chemical bond vectors adjacent to the graphical chemical bond vector, $\theta$ is calculated respectively for each adjacent graphical chemical bond vector and the minimum one among all the calculated $\theta$ is selected as the rotating angle.

For example, assuming that the graphical chemical bond vector to be adjusted is $a$ and two graphical chemical bond vectors $b$ and $c$ adjacent to $a$ are located on the left and right sides of $a$. If the rotating direction is determined as clockwise, then $b$ and the vector obtained from rotating $a$ by $\theta_1$ in the clockwise direction may compose a regular $N_1$- sided polygon; and $c$ and the vector obtained from rotating bond $a$ by $\theta_2$ in the clock-

wise direction may compose a regular $N_2$- sided polygon. If $\theta_1 < \theta_2$, according to the preferred embodiment, $\theta_1$ (equivalent to a recommendation angle) is set as $\theta$.

Preferably, $N$ is less than or equal to 10. In the practice, the shape of the molecule ring may be regular polygon, such as regular triangle, regular octagon, and even regular 10- sided polygon. Therefore, the preferred embodiment complies with physical laws, and then mathematic expectation to operations of the users can be implemented better.

Preferably, in step S30, if the end at which the typesetting focus is located is not connected to ends of other graphical chemical bond vectors and a current angle between the graphical chemical bond vector and an adjacent graphical chemical vector is beyond an angle range, the rotation angle based on each received input is set as one minimum unit for rotation. In the case that the angle to be adjusted is beyond the range of interior angle from the regular triangle to the regular 10- sided polygon, it is difficult to mathematically anticipate the angle to be adjusted. Thus, it is preferred to respond to user's operation by the stepless adjustment.

Preferably, the method further comprises at least one of the following steps: prompting value of an angle between the graphical chemical bond vector and its adjacent graphical chemical bond vector; and prompting the value and number of sides of a regular polygon simultaneously if the value is equal to that of an interior angle in the regular polygon.

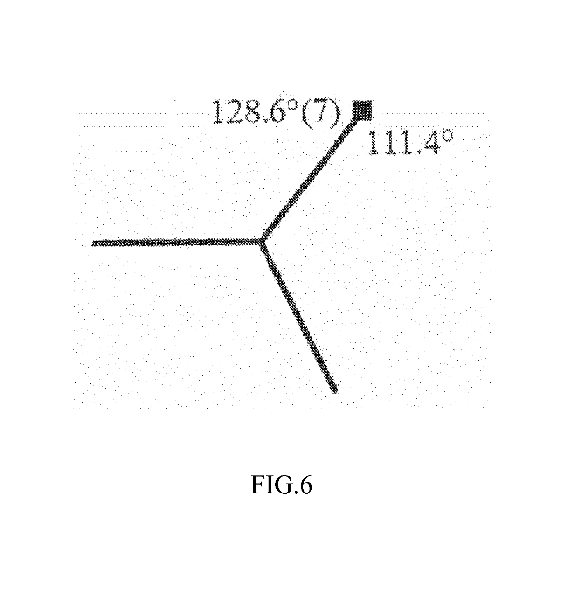

Figs. 5 and 6 are schematic diagrams of prompt angles according to preferred embodiments of the present application. As shown in Figs. 5 and 6, angle values shown on both sides of the vector to be rotated represent the angle between it and its adjacent vector (s). If one angle value is equal to an interior angle of one regular polygon, the number of sides of the polygon is also shown next to the angle value. In the prior art, no prompt has been provided for rotation of the graphical chemical bond vectors. The more sides a ring-shaped structure has, the closer value of the angle is, and thus it is difficult for the user to determine the angle value or required shape of the graphical chemical bond vector. However, according to the preferred embodiment, the user can understand easily whether the prompted structure is the desired one.

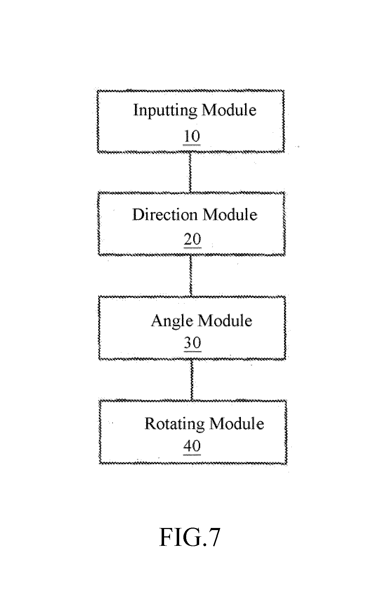

Fig. 7 is a schematic diagram illustrating an apparatus for adjusting angles between graphical chemical bond vectors according to an embodiment of the present application. The apparatus comprises an inputting module 10, a direction module 20, an angle module 30 and a rotating module 40. The inputting module 10 is configured to receive an input from specific keys of a keyboard when a typesetting focus is located in one end of a graphical chemical bond vector. The direction module 20 is configured to determine a rotating direction for the graphical chemical bond vector based on the received input, and the angle module 30 is configured to set a rotating angle of the graphical chemical bond vector in the determined rotating direction. The rotating module 40 is configured to rotate the graphical chemical bond vector about another end thereof by the set rotating angle and in the determined rotating direction.

The usability and efficiency of chemical typesetting can be improved by the apparatus.

Preferably, the angle module 30 may comprise a stepless module. If the stepless module determines that the end at which the typesetting focus is located is further connected to ends of other graphical chemical bond vectors, the stepless module then operates to set, based on each received input, 1) the rotation angle for the vector as a minimum unit for rotation, and 2) angles and lengths for other vectors, which are required to keep connection between the graphical chemical bond vector and the other vectors. If the stepless module determines that the end at which the typesetting focus is located is not connected to ends of other vectors and a current angle between the graphical chemical bond vector and an adjacent graphical chemical bond vector is beyond an angle range, then the stepless module operates to set the rotation angle based on each received input as a minimum unit for rotation.

the angle module 30 may further comprise a step module configured to determine that the end at which the typesetting focus is located is not connected to ends of other graphical chemical bond vectors and a current angle between the graphical chemical bond vector and an adjacent graphical chemical bond vector is within an angle range, and then set the rotation angle based on each received input as:

$$\theta = \alpha - \beta,$$

where $\alpha$ is an interior angle of a regular N-sided polygon consisted of: 1) a graphical chemical bond vector which is formed by rotating the graphical chemical bond vector by the minimum angle in the rotating direction, and 2) its adjacent graphical chemical bond vector, and $\beta$ is the current angle between the graphical chemical bond vector and the adjacent graphical chemical bond vector.

Preferably, the apparatus may further comprise at least one of the following modules: a first prompting module configured to prompt value of an angle between the graphical chemical bond vector and its adjacent graphical chemical bond vectors; and a second prompting module configured to prompt the value and number of sides of a regular polygon simultaneously if the value is equal to that of an interior angle in the regular polygon.

According to the preferred embodiments of the present application, angle adjustment can be performed by the combination of the stepless and step modules and the user can be provided with the prompts obviously to facilitate the user to use the keyboard to perform angle adjustment for graphical chemical bond vectors.

According to the above embodiments of the present invention, the user can rotate graphical chemical bond vectors flexibly and quickly so that efficiency of typesetting can be improved and learning difficulty can be reduced.

It will be readily apparent to those skilled in the art that each of modules or steps as discussed in the above may be implemented with a common computer device. In addition, the modules or steps of the present application may be concentrated in a single computer device or distributed in a network composed of multiple computer devices. Optionally, the modules or steps may be achieved by using codes of executable programs, so that they can be stored in a storage medium, or can be respectively fabricated into various individual integrated circuit modules, or the plurality of the modules or steps can be fabricated into one individual integrated circuit module. Therefore, the present application is not limited to any particular hardware, software or combination thereof.

The foregoing is only preferred embodiments of the present application, and it is not intended to limit the present application. Moreover, it will be apparent to those skilled in the art that various modifications and variations can be made to the present application. Thus, any modifications, equivalent substitutions, improvements etc. within the spirit and principle of the present application should be included within the scope of protection of the application.

**Claims**

1. A method for adjusting angles between graphical chemical bond vectors comprising:

     receiving an input from specific keys of a keyboard when a typesetting focus is located at one end of a graphical chemical bond vector;
     determining a rotating direction for the vector based on the received input;
     setting a rotating angle for the vector in the determined rotating direction; and
     rotating the vector about the other end thereof by the set rotating angle and in the determined rotating direction.

2. The method according to claim 1, wherein the determining comprises:

     determining that the received input is received from a first key, and then it is determined that the rotating direction is clockwise; or
     determining that the received input is received from a second key, and then it is determined that the rotating direction is anticlockwise.

3. The method according to claim 1, wherein the determining comprises:

> determining that the received input is received from a first key and angle to be adjusted for the graphical chemical bond vector is within a threshold range, and
> then it is determined that the rotating direction is clockwise;

> > determining that the received input is received from the first key and the angle to be adjusted for the graphical chemical bond vector is beyond the threshold range, and then it is determined that the rotating direction is anticlockwise;
> > determining that the received input is received from a second key and the angle to be adjusted for the graphical chemical bond vector is within a threshold range, and then it is determined that the rotating direction is anticlockwise; and
> > determining that the received input is received from the second key and the angle to be adjusted for the graphical chemical bond vector is beyond the threshold range, and then it is determined that the rotating direction is clockwise.

4. The method according to claim 1, wherein during the setting, if said one end is connected to ends of other graphical chemical bond vectors, the rotation angle is set as a minimum unit for rotation based on each received input, and angles and lengths for other vectors, which are required to keep connection between all the connected vectors, are set.

5. The method according to claim 1, wherein, during the step of setting, if said one end is not connected to other graphical chemical bond vectors and a current angle between the graphical chemical bond vector and an adjacent graphical chemical bond vector is within an angle range, the rotation angle based on each received input is set by rule of

$$\theta = \alpha - \beta,$$

where $\alpha$ is an interior angle of a regular N-sided polygon consisted of: 1) a graphical chemical bond vector which is formed by rotating the graphical chemical bond vector by a minimum angle in the rotating direction and 2) its adjacent graphical chemical bond vector, and
$\beta$ is the current angle between the graphical chemical bond vector and the adjacent graphical chemical bond vector.

6. The method according to claim 5, wherein when there are a plurality of graphical chemical bond vectors adjacent to the graphical chemical bond vector, $\theta$ is calculated respectively for each adjacent graphical chemical bond vector and the minimum one among all the calculated $\theta$ is selected as the rotating angle.

7. The method according to claim 5, wherein $N$ is lower than or equal to 10.

8. The method according to claim 1, wherein,
if said one end is not connected to ends of other graphical chemical bond vectors and a current angle between the graphical chemical bond vector and an adjacent graphical chemical bond vector is beyond an angle range, the step of setting comprises:

> setting the rotation angle based on each received input as a minimum unit for rotation.

9. The method according to claim 1, further comprising at least one of the following steps:

> prompting value of an angle between the graphical chemical bond vector and its adjacent graphical chemical bond vector; and
> prompting the value and number of sides of a regular polygon simultaneously if the value is equal to that of an interior angle in the regular polygon.

10. An apparatus for adjusting angles between graphical chemical bond vectors comprising:

> an inputting module configured to receive an input from specific keys of a keyboard when a typesetting focus is located at one end of a graphical chemical bond vector;
> a direction module configured to determine a rotating direction for the graphical chemical bond vector based on the received input;
> an angle module configured to set a rotating angle for the graphical chemical bond vector in the determined rotating direction; and
> a rotating module configured to rotate the graphical chemical bond vector about the other end thereof by the set rotating angle and in the determined rotating direction.

11. The apparatus according to claim 10, wherein the angle module comprises:

> a stepless module configured, based on each received input, to
> set the rotation angle as a minimum unit for rotation, and set angles and lengths for other graphical chemical bond vectors, which are required to keep connection between the graphical chemical bond vector and the other graphical chemical bond vectors, if said one end is further connected to ends of other graphical chemical bond vectors; or

set the rotation angle as a minimum unit for rotation, if said one end is not connected to ends of other graphical chemical bond vectors and a current angle between the graphical chemical bond vector and an adjacent graphical chemical bond vector is beyond an angle range; and

a step module configured to, if said one end is not connected to ends of other graphical chemical bond vectors and a current angle between the graphical chemical bond vector and an adjacent graphical chemical bond vector is within an angle range, set the rotation angle based on each received input as:

$$\theta = \alpha - \beta,$$

where $\alpha$ is an interior angle of a regular N-sided polygon consisted of: 1) a graphical chemical bond vector which is formed by rotating the graphical chemical bond vector by the minimum angle in the rotating direction and 2) its adjacent graphical chemical bond vector, and

$\beta$ is the current angle between the graphical chemical bond vector and the adjacent graphical chemical bond vector.

12. The apparatus according to claim 10, further comprising at least one of the following modules:

a first prompting module configured to prompt value of an angle between the graphical chemical bond vector and its adjacent graphical chemical bond vectors; and

a second prompting module configured to prompt the value and number of sides of a regular polygon simultaneously if the value is equal to that of an interior angle of the regular polygon.

FIG.1

FIG.2

Receiving an input from specific keys of a keyboard when a typesetting focus is located at one end of a graphical chemical bond vector — S 10

Determining a rotating direction for the vector based on the received input — S 20

Setting a rotating angle for the vector in the determined rotating direction — S 30

Rotating the vector about the other end thereof by the set rotating angle and in the determined rotating direction — S 40

FIG.3

FIG.4

FIG.5

FIG.6

Inputting Module
10

Direction Module
20

Angle Module
30

Rotating Module
40

FIG.7

# INTERNATIONAL SEARCH REPORT

| International application No. |
| --- |
| **PCT/CN2011/083394** |

**A. CLASSIFICATION OF SUBJECT MATTER**

G06F 17/21(2006.01)i

According to International Patent Classification (IPC) or to both national classification and IPC

**B. FIELDS SEARCHED**

Minimum documentation searched (classification system followed by classification symbols)

IPC: G06F 17/-; G06F3/-; G06T15/-;

Documentation searched other than minimum documentation to the extent that such documents are included in the fields searched

Electronic data base consulted during the international search (name of data base and, where practicable, search terms used)

CNPAT, CNKI,WPI,EPODOC: image?, edit+, key?, chemic+, rotat+, roll+, clock, adjust+, draw+, direction, plot+

**C. DOCUMENTS CONSIDERED TO BE RELEVANT**

| Category* | Citation of document, with indication, where appropriate, of the relevant passages | Relevant to claim No. |
| --- | --- | --- |
| Y | JP11109940 A (CANON KK) 23 Apr. 1999 (23.04.1999) description paragraph 13- paragraph 38 | 1-3, 10 |
| A | | 4-9, 11,12 |
| Y | CN1477892A (LG ELECTRONICS INC)    25 Feb. 2004 (25.02.2004) description page 2 line 1- page 6 line 1 | 1-3, 10 |
| A | | 4-9, 11,12 |
| A | CN101178815 A (UNIV ELECTRONIC SCI & TECHNOLOGY) 14 May 2008 (14.05.2008) the whole document | 1-12 |

☐ Further documents are listed in the continuation of Box C.         ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 14 Jan. 2012（14.01.2012） | 08 Mar. 2012 (08.03.2012) |

| Name and mailing address of the ISA State Intellectual Property Office of the P. R. China No. 6, Xitucheng Road, Jimenqiao Haidian District, Beijing 100088, China Facsimile No. (86-10) 62019451 | Authorized officer YU, Chen Telephone No. (86-10) **62411716** |
| --- | --- |

Form PCT/ISA /210 (second sheet) (July 2009)

### INTERNATIONAL SEARCH REPORT
Information on patent family members

| International application No. |
| :--- |
| **PCT/CN2011/083394** |

| Patent Documents referred in the Report | Publication Date | Patent Family | Publication Date |
| :---: | :---: | :---: | :---: |
| JP11109940 A | 23.04.1999 | None | |
| CN1477892 A | 25.02.2004 | US2004008218A1 | 15.01.2004 |
| | | KR20040005291A | 16.01.2004 |
| | | KR100608735B1 | 04.08.2006 |
| | | CN100423595C | 01.10.2008 |
| CN101178815 A | 14.05.2008 | None | |

Form PCT/ISA /210 (patent family annex) (July 2009)